# EUROPEAN PATENT APPLICATION

(11) **EP 4 757 344 A1**
(43) Date of publication of application: **10.06.2026**
(21) Application number: 25213330.1
(22) Date of filing: 04.11.2025
(51) Int. Cl.: H04R 25/00

(54) **HEARING AID EAR LOCK**

(30) Priority: 06.12.2024 EP 24218080
(71) Applicant: Oticon A/S, 2765 Smørum (DK)
(72) Inventor: MOOSMAYER, Rainer, DK-2765 Smørum (DK)
(74) Representative: Demant

(57) **Abstract**

An ear lock for maintaining an output transducer of a hearing aid in a user's ear canal, the ear lock comprising a body comprising an aperture extending along a longitudinal axis of the body, the body configured to receive an output transducer of a hearing aid within the aperture, wherein the body is formed from an elastic material; a first extension attached to the body on a first side of the body and extending away from the body, wherein the first extension is configured to abut a first surface of the ear canal; and a second extension attached to the body on a second side of the body and extending away from the body, wherein the second extension is configured to abut a second surface of the ear canal.

## Description

### TECHNICAL FIELD

The present application relates generally to the field of hearing aids. In particular, the present application relates generally to ear locks for hearing aids.

### SUMMARY

Ear locks for speaker units are used to assure and maintain the proper placement of the output transducer in a user's ear canal even when the ear canal deforms during chewing, sport activities, etc. Previously known ear locks have many drawbacks to them. For example, known ear locks are long and typically extend out of the user's ear canal. Therefore, current ear locks are visible in the ear due to their size and form factor. Further, known ear locks use relatively stiff material which causes irritation on the user's skin. Known ear locks can therefore be uncomfortable for a user, and in certain situations the ergonomic fit does not work for the ear lock.

### SUMMARY

In certain aspects of the present application, an ear lock for a hearing aid is disclosed. The ear lock can be configured for maintaining an output transducer of a hearing aid in a user's ear canal. The ear lock can include a body. The body can comprise an aperture. The aperture can extend along a longitudinal axis of the body. The body is configured to receive an output transducer of a hearing aid within the aperture. The body can be formed from an elastic material. The ear lock can include a first extension. The first extension can be attached to the body on a first side of the body. The first extension can extend away from the body. The first extension can be configured to about a first surface of the ear canal. The ear lock can include a second extension. The second extension can be attached to the body on a second side of the body. The second extension can extend away from the body. The second extension can be configured to about a second surface of the ear canal.

Thereby, an improved ear lock for a hearing aid is disclosed. Advantageously, the disclosed ear locks can be much smaller. For example, they can fit entirely with the ear canal. Therefore, the ear lock is almost invisible to a third person. Further, the specific construction and/or materials allows for a softer ear lock, thereby improving comfort to a user. The design of the disclosed ear locks can also fit within all types of ear canals. The design of the disclosed ear lock can retain the output transducer in the ear canal better than known ear locks, including in situations where the ear canal deforms during chewing, sport activities, etc. Moreover, the disclosed embodiments of ear locks are advantageous as they do not require the dome of a hear aid to act as an anchor.

Further disclosed is a hearing aid including embodiments of the ear lock disclosed herein.

Further disclosed is an output transducer including embodiments of the ear lock disclosed herein.

### Ear lock

Disclosed herein is an ear lock (e.g., ear lock device, receiver lock, sport lock, hearing aid lock, retention, hearing aid retainer, hearing aid device, accessory device). The ear lock is for maintaining (e.g., preventing release, preventing falling out, retaining, holding in, keeping stable) an output transducer of a hearing aid in a user's ear canal, such as during ordinary use. The ear lock is configured to maintain an output transducer of a hearing aid in a user's ear canal.

The hearing aid can be a behind-the-ear (BTE) hearing aid. The hearing aid can be an in-the-ear (ITE) hearing aid. The hearing aid can be a Receiver in the ear (RITE) hearing aid.

The output transducer can be understood to be, for example, a speaker (e.g., speaker unit). The output transducer can be understood to be, for example, a receiver (e.g., receiver unit). The output transducer can be fully within an ear canal of the user during use. In certain embodiments, electrical connections between the output transducer and further components of the hearing aid can be used. For example, the output transducer can be electrically connected to a behind-the-ear unit of a BTE hearing aid.

In one or more example ear locks, the ear lock includes a body. The body can have an outer surface. The outer surface can define the outer bounds of the body. The body can have an inner surface. The inner surface can define an aperture. The body can have a thickness extending between the outer surface and the inner surface. The thickness can be, for example, from 0.3-0.9mm. The thickness can be 0.7mm (or about 0.7mm).

The body can have a rectangular (or generally rectangular) cross-section. The body can have a square (or generally square) cross-section. The body can have a circular (or generally circular) cross section. The body can be ring-shaped. The body can be a tube. The body can be a rectangular tube. The particular shape of the body is not limiting.

The body can have a length (L) in a direction along the longitudinal axis. The body can have width (W) in a direction perpendicular to the longitudinal axis. The body can have a height (H) in a direction perpendicular to the longitudinal axis and the longitudinal axis. As an example, the body can have a width of 5-6mm. The body can have a length of 2-3mm. The body can have a height of 4-5mm. As an example, the body can have a length of 2.2mm, a width of 5.1mm, and a height of 4.6mm.

The body can have a first side. The body can have a second side. The first side can be understood as a first half of the body. The second side can be understood as a second half of the body opposite the first half. The first side and the second side can be defined with respect to one another by a plane extending from the longitudinal axis and parallel to the longitudinal axis.

In one or more example ear locks, the outer surface can have a generally rectangular shape. For example, the outer surface can have a first surface and a second surface generally opposite the first surface. The outer surface can have a third surface and a fourth surface generally opposite the third surface. The first surface can be connected to the third surface and the fourth surface via a first and second corner, respectively. The second surface can be connected to the third surface and the fourth surface via a third and fourth corner, respectively. The first, second, third, and fourth corners can be rounded. The first, second, third, and fourth corners can be angled.

The body can include an aperture (e.g., hole, gap, opening). The inner surface of the body can define the aperture. The aperture can pass fully through the body. For example, the aperture can pass from a first longitudinal end to a second longitudinal end of the body. The first longitudinal end can be opposite the second longitudinal end. The aperture can extend along a longitudinal axis of the body. The longitudinal axis can extend between the first longitudinal end and the second longitudinal end.

The aperture can have a rectangular (or general rectangular) cross-section. In other words, an inner surface of the body can have a rectangular (or generally rectangular) shape. For example, the aperture can have side lengths of 3-4mm by 3-4mm, such as 3.7mm by 3.2mm. The aperture can have a square (or generally square) cross-section. The aperture can have a circular (or generally circular) cross section. For example, the aperture can have a diameter of 3-4mm. The aperture can be shaped generally similar to the output transducer. The particular shape of the aperture is not limiting.

In certain example ear locks, the aperture can make up 50, 60, 70, 80, or 90% of a cross-section of the body of the ear lock.

The body is formed from an elastic and/or flexible and/or resilient material. In other words, the body is configured to stretch and then return to an unstretched configuration (e.g., shape). In this way, the aperture can be smaller than the output transducer. The body (and thus the aperture) can stretch to receive the output transducer within the aperture. When the body (and thus the aperture) returns to its unstretched configuration, the body can hold the output transducer in place. For example, the body can hold the output transducer in place within the aperture.

An elastic material is a material that can stretch or compress under stress and return to their original form once the stress is removed. The body can be formed of an elastic material. The first extension can be formed of an elastic material. The second extension can be formed of an elastic material.

The body can have the same elasticity as the first extension. The body can have the same elasticity as the second extension. The body can have a lower elasticity than the first extension. The body can have a lower elasticity than the second extension.

In one or more example ear locks, the body is configured to stretch to receive the output transducer within the aperture. The ear lock can be configured to be removable from the output transducer. The ear lock can be permanently attached to the output transducer.

In one or more example ear locks, the body includes a first section where an outer surface of the body is aligned along the longitudinal axis. In one or more example ear locks, the body includes a second section where the outer surface curves with respect to the longitudinal axis. In one or more example ear locks, the body includes a third section where the outer surface curves with respect to the longitudinal axis. In one or more example ear locks, the body includes a third section where the outer surface curves with respect to the longitudinal axis.

The second section and the third section can be longitudinal ends of the body. The second section can end at the first longitudinal end of the body. The third section can end at a second longitudinal end of the body. The first section can be longitudinally between the second section and the third section. The first section can be flat. The second section and the third section can curve towards the longitudinal axis. The second section and the first section can be mirrors of one another.

In one or more example ear locks, the ear lock includes a first extension. The first extension can be formed from an elastic and/or flexible and/or resilient material. The first extension can be compressible. The first extension can be configured to about a first surface of the ear canal. The first extension can be configured to about a first surface of the ear canal during use of the hearing aid. The first surface can be considered an internal surface of the user.

The first extension can be configured to be compressed by the first surface. For example, during use, the first extension can be compressed by the first surface of the ear canal. The first extension wants to return to its original shape, and therefore provides a force on the first surface. This can help maintain the ear lock in place.

The first extension can be attached to the body. For example, the first extension can be integrally formed with the body. The first extension could be adhered to the body. The first extension could be attached to the body during and/or after manufacturing of the body. The first extension can be attached on a first side of the body. The first extension can be attached on a first surface of the body.

The first extension can extend away from the body. In other words, the first extension can extend radially away from the body. The first extension can extend away from the longitudinal axis. The first extension can extend away from the outer surface of the body. The first extension can be additionally understood as a first arm. In one or more example ear locks, the first extension extends orthogonal to the longitudinal axis. The first extension can be fully located along a longitudinal direction between the first longitudinal end and the second longitudinal end of the body.

In one or more example ear locks, the first extension comprises a plurality of arms. In one or more example ear locks, the first extension comprises a first arm. In one or more example ear locks, the first extension comprises a second arm. In one or more example ear locks, the first extension comprises a bridge arm (e.g., third arm, support arm, extension arm). In one or more example ear locks, first extension comprises a first arm, a second arm, and a bridge arm connecting the first arm and the second arm.

For example, the first extension can include three interconnected arms. The first arm and the second arm can extend radially away from the body. The first arm and the second arm can extend radially away from the first surface of the body. The first arm and the second arm can extend radially away from the first corner and the second corner of the body, respectively.

The first arm can be connected to the body at a first proximal end. The first arm can extend from the first proximal end to a first distal end. The second arm can be connected to the body at a second proximal end. The second arm can extend from the second proximal end to a second distal end.

The bridge arm can connect the first distal end and the second distal end. The bridge arm can extend between the first distal end and the second distal end. The bridge arm can be straight. The bridge arm can be curved. The bridge arm can be an arc. The bridge arm can be concave with respect to the body.

In certain examples, the first arm, the second arm, and the bridge arm can define an extension aperture. The extension aperture can extend along the longitudinal axis. The extension aperture can extend in the same direction as the aperture. In certain examples, the bridge arm is connected to the body. In this embodiment, no extension aperture is formed.

In certain examples, the first extension does not include a bridge arm. Therefore, the first extension can include the first arm and the second arm. The first extension can include an additional arm having a proximal end attached to the body and a free end spaced radially away from the body.

In one or more example ear locks, the first extension comprises a single arm. In one or more example ear locks, the first extension and the second extension are the same type of extension. For example, the second extension can be the first extension but on a second side.

In one or more example ear locks, the ear lock includes a second extension. The second extension an be an elastic and/or flexible and/or resilient material. The second extension can be compressible. The second extension can be configured to abut a second surface of the ear canal. The second extension can be configured to abut a second surface of the ear canal during use of the hearing aid. The second surface can be considered an internal surface of the user. The second surface of the ear canal is different from the first surface of the ear canal.

The second extension can be configured to be compressed by the second surface. For example, during use, the second extension can be compressed by the second surface of the ear canal. The second extension wants to return to its original shape, and therefore provides a force on the second surface. This can help maintain the ear lock in place.

When inserted into an ear canal (e.g., during use), both the first extension and the second extension can be compressed by the ear canal. As the first extension and the second extension are both elastic, they desire to return to their original shape. Therefore, the first extension and the second extension provide an outward force on the ear canal. As the first extension and the second extension are on different sides of the ear lock, the forces help to retain the ear lock within the ear canal.

The second extension can be attached to the body. For example, the second extension can be integrally formed with the body. The second extension could be adhered to the body. The second extension could be attached to the body during and/or after manufacturing of the body. The second extension can be attached on a second side of the body. The second extension can be attached on a second surface of the body.

The second extension can extend away from the body. In other words, the second extension can extend radially away from the body. The second extension can extend away from the longitudinal axis. In other words, the second extension can extend longitudinally away from the body. The second extension can extend away from the outer surface of the body. The second extension can be additionally understood as a second arm. The second extension can be understood as a deflection arm.

In one or more example ear locks, the first side is generally opposite on the body from the second side. In one or more example ear locks, the first side is opposite on the body from the second side. In other words, the first extension and the second extension extend from opposite sides of the body.

In certain examples, the first extension is attached to the body on the first surface of the body. In certain examples, the first extension is attached to the body on the first surface of the body and/or the first corner and/or the second corner of the body.

In certain examples, the second extension is attached to the body on the second surface of the body.

In one or more example ear locks, the second extension is curved with respect to the longitudinal axis. In one or more example ear locks, the second extension extends at least partially away from the longitudinal axis. In one or more example ear locks, the second extension extends at least partially in the direction of the longitudinal axis. The second extension can have a length of, for example, 8-9mm. In certain examples, the second extension can have a length of 8.2mm.

In other words, the second extension extends radially away from the body and longitudinally away from the body. For example, the second extension can extend towards an exit of the ear canal during use. The second extension can be attached to the body at a second proximal end. In certain examples, the second extension is not fully located along a longitudinal direction between the first longitudinal end and the second longitudinal end of the body. For example, the second extension can have a second distal end. The second distal end can be considered a free end. The second distal end may be spaced longitudinally away from the body. The second extension can include a second extension body extending between the second distal end and the second proximal end.

In certain examples, the second extension may be straight. The second extension may be shaped like an arc. The second extension may be arcuate. The second extension may be a concave arc with respect to the longitudinal axis.

The first extension can be configured to be flexible. The first extension can be configured to be compressible. For example, the first extension can be configured to at least partially collapse to reduce the size of the extension aperture.

The second extension can be configured to be flexible. The second extension can be configured to be compressible. For example, second first extension can be configured to compress towards the longitudinal axis.

In one or more example ear locks, the second extension is configured to apply a spring effect force on the second surface. In one or more example ear locks, the second extension is configured to press the first extension into the first surface.

In other words, the second extension is configured to be compressed in order to apply an opposite force. For example, during insertion into the ear canal, the second extension is compressed by the second surface.

While the user is inserting the output transducer with the ear lock into the ear canal, the second extension will be compressed (e.g., deflected) by the shape of the ear canal. This deflection creates a spring effect. In certain examples, the second extension will create a force resulting the opposite side of the ear lock to be pressed towards the ear canal.

In one or more example ear locks, the second extension is configured to apply an inward force into the ear canal. The second extension is configured to apply a longitudinally inward force into the ear canal. The second extension is configured to apply a radially inward force. In certain examples, the second extension can prevent accidental removal of the ear lock by applying the inward force. Because of the shape of the second extension of the ear lock, the deflection of the second extension will create a force that will push the ear lock (and any connected output transducer) into the ear canal. This prevents an unintended displacement of the output transducer to the outside of the ear canal.

In one or more example ear locks, the ear lock is configured to be centered in the ear canal by a three-point suspension with respect to the first extension and the second extension. In one or more example ear locks, the ear lock is configured to be centered in the ear canal by the first extension and the second extension. The first extension and the second extension can be compressed when the ear lock is inserted into the ear canal, and can center the ear lock. This can allow for the ear lock to center the output transducer in the ear canal. The first extension may have a first arm and a second arm, resulting in two points of the three-point suspension system, along with the single second extension. In one or more example ear locks, the ear lock is configured to be centered in the ear canal by a two-point suspension with respect to the first extension and the second extension.

In one or more example ear locks, the first extension and the second extension are configured to prevent rotation of the ear lock in the ear canal. In one or more example ear locks, the first extension and the second extension are configured to prevent rotation of the output transducer in the ear canal. For example, pressure and/or surface friction of the first extension and/or the second extension with the ear canal can prevent rotation.

The ear lock can be configured to fit fully within an ear canal of the user. In one or more example ear locks, the ear lock does not extend out of the ear canal during use. This can advantageously keep the ear lock discreet.

In certain examples, the body, the first extension, and the second arm are made of the same material. In one or more example ear locks, the body is formed from a first material and the first extension and the second extension are formed from a second material. In one or more example ear locks, the body is formed from a first material, the first extension is formed from a second material, and the second extension is formed from a third material. The first material may be less elastic (e.g., less compressible) than the second material. The first material can have a different Shore Hardness than the second material (and optionally the third material. The first material can have a higher Shore Hardness than the second material (and optionally the third material). The second material can be overmolded on the first material.

In one or more example ear locks, the first material comprises a polyamide. In one or more example ear locks, the second material comprises silicon.

In one or more example ear locks, the first material comprises an ester-based thermoplastic polyurethane. In one or more example ear locks, the second material comprises silicon.

### Hearing aid

The hearing aid may be adapted to provide a frequency dependent gain and/or a level dependent compression and/or a transposition (with or without frequency compression) of one or more frequency ranges to one or more other frequency ranges, e.g. to compensate for a hearing impairment of a user. The hearing aid may comprise a signal processor for enhancing the input signals and providing a processed output signal.

The hearing aid may comprise an output transducer (e.g., output unit) for providing a stimulus perceived by the user as an acoustic signal based on a processed electric signal. The output transducer may a vibrator of a bone conducting hearing aid. The output transducer may comprise an output unit. The output transducer may comprise a receiver (loudspeaker) for providing the stimulus as an acoustic signal to the user (e.g. in an acoustic (air conduction based) hearing aid). The output transducer may comprise a vibrator for providing the stimulus as mechanical vibration of a skull bone to the user (e.g. in a bone-attached or bone-anchored hearing aid). The output transducer may (additionally or alternatively) comprise a (e.g. wireless) transmitter for transmitting sound picked up-by the hearing aid to another device, e.g. a far-end communication partner (e.g. via a network, e.g. in a telephone mode of operation).

The hearing aid may comprise an input unit for providing an electric input signal representing sound. The input unit may comprise an input transducer, e.g. a microphone, for converting an input sound to an electric input signal. The input unit may comprise a wireless receiver for receiving a wireless signal comprising or representing sound and for providing an electric input signal representing said sound.

The wireless receiver and/or transmitter may e.g. be configured to receive and/or transmit an electromagnetic signal in the radio frequency range (3 kHz to 300 GHz). The wireless receiver and/or transmitter may e.g. be configured to receive and/or transmit an electromagnetic signal in a frequency range of light (e.g. infrared light 300 GHz to 430 THz, or visible light, e.g. 430 THz to 770 THz).

The hearing aid may comprise a directional microphone system adapted to spatially filter sounds from the environment, and thereby enhance a target acoustic source among a multitude of acoustic sources in the local environment of the user wearing the hearing aid. The directional system may be adapted to detect (such as adaptively detect) from which direction a particular part of the microphone signal originates. This can be achieved in various different ways as e.g. described in the prior art. In hearing aids, a microphone array beamformer is often used for spatially attenuating background noise sources. The beamformer may comprise a linear constraint minimum variance (LCMV) beamformer. Many beamformer variants can be found in literature. The minimum variance distortionless response (MVDR) beamformer is widely used in microphone array signal processing. Ideally the MVDR beamformer keeps the signals from the target direction (also referred to as the look direction) unchanged, while attenuating sound signals from other directions maximally. The generalized sidelobe canceller (GSC) structure is an equivalent representation of the MVDR beamformer offering computational and numerical advantages over a direct implementation in its original form.

The hearing aid may comprise antenna and transceiver circuitry allowing a wireless link to an entertainment device (e.g. a TV-set), a communication device (e.g. a telephone), a wireless microphone, a separate (external) processing device, or another hearing aid, etc. The hearing aid may thus be configured to wirelessly receive a direct electric input signal from another device. Likewise, the hearing aid may be configured to wirelessly transmit a direct electric output signal to another device. The direct electric input or output signal may represent or comprise an audio signal and/or a control signal and/or an information signal.

The hearing aid may be constituted by or form part of a portable (i.e. configured to be wearable) device, e.g. a device comprising a local energy source, e.g. a battery, e.g. a rechargeable battery. The hearing aid may e.g. be a low weight, easily wearable, device, e.g. having a total weight less than 100 g, such as less than 20 g, such as less than 5 g.

The hearing aid may comprise a 'forward' (or 'signal') path for processing an audio signal between an input and an output of the hearing aid. A signal processor may be located in the forward path. The signal processor may be adapted to provide a frequency dependent gain according to a user's particular needs (e.g. hearing impairment). The hearing aid may comprise an 'analysis' path comprising functional components for analyzing signals and/or controlling processing of the forward path. Some or all signal processing of the analysis path and/or the forward path may be conducted in the frequency domain, in which case the hearing aid comprises appropriate analysis and synthesis filter banks. Some or all signal processing of the analysis path and/or the forward path may be conducted in the time domain.

An analogue electric signal representing an acoustic signal may be converted to a digital audio signal in an analogue-to-digital (AD) conversion process, where the analogue signal is sampled with a predefined sampling frequency or rate fₛ, fₛ being e.g. in the range from 8 kHz to 48 kHz (adapted to the particular needs of the application) to provide digital samples xₙ (or x[n]) at discrete points in time tₙ (or n), each audio sample representing the value of the acoustic signal at tₙ by a predefined number N_{b} of bits, N_{b} being e.g. in the range from 1 to 48 bits, e.g. 24 bits. Each audio sample is hence quantized using N_{b} bits (resulting in 2^{Nb} different possible values of the audio sample). A digital sample x has a length in time of 1/fₛ, e.g. 50 µs, for *fₛ* = 20 kHz. A number of audio samples may be arranged in a time frame. A time frame may comprise 64 or 128 audio data samples. Other frame lengths may be used depending on the practical application.

The hearing aid may comprise an analogue-to-digital (AD) converter to digitize an analogue input (e.g. from an input transducer, such as a microphone) with a predefined sampling rate, e.g. 20 kHz. The hearing aids may comprise a digital-to-analogue (DA) converter to convert a digital signal to an analogue output signal, e.g. for being presented to a user via an output transducer.

The hearing aid, e.g. the input unit, and or the antenna and transceiver circuitry may comprise a transform unit for converting a time domain signal to a signal in the transform domain (e.g. frequency domain or Laplace domain, Z transform, wavelet transform, etc.). The transform unit may be constituted by or comprise a TF-conversion unit for providing a time-frequency representation of an input signal. The time-frequency representation may comprise an array or map of corresponding complex or real values of the signal in question in a particular time and frequency range. The TF conversion unit may comprise a filter bank for filtering a (time varying) input signal and providing a number of (time varying) output signals each comprising a distinct frequency range of the input signal. The TF conversion unit may comprise a Fourier transformation unit (e.g. a Discrete Fourier Transform (DFT) algorithm, or a Short Time Fourier Transform (STFT) algorithm, or similar) for converting a time variant input signal to a (time variant) signal in the (time-)frequency domain. The frequency range considered by the hearing aid from a minimum frequency fₘᵢₙ to a maximum frequency fₘₐₓ may comprise a part of the typical human audible frequency range from 20 Hz to 20 kHz, e.g. a part of the range from 20 Hz to 12 kHz. Typically, a sample rate fₛ is larger than or equal to twice the maximum frequency fₘₐₓ, fₛ ≥ 2fₘₐₓ. A signal of the forward and/or analysis path of the hearing aid may be split into a number *NI* of frequency bands (e.g. of uniform width), where *NI* is e.g. larger than 5, such as larger than 10, such as larger than 50, such as larger than 100, such as larger than 500, at least some of which are processed individually. The hearing aid may be adapted to process a signal of the forward and/or analysis path in a number *NP* of different frequency channels *(NP* ≤ *NI).* The frequency channels may be uniform or non-uniform in width (e.g. increasing in width with frequency), overlapping or non-overlapping.

The hearing aid may further comprise other relevant functionality for the application in question, e.g. compression, noise reduction, etc.

The hearing aid may comprise a hearing instrument, e.g. a hearing instrument adapted for being located at the ear or fully or partially in the ear canal of a user.

### Definitions:

In the present context, a hearing aid, e.g. a hearing instrument, refers to a device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. Such audible signals may e.g. be provided in the form of acoustic signals radiated into the user's outer ears and/or acoustic signals transferred as mechanical vibrations to the user's inner ears through the bone structure of the user's head and/or through parts of the middle ear.

The hearing aid may be configured to be worn in any known way, e.g. as a unit arranged behind the ear with a tube leading radiated acoustic signals into the ear canal or with an output transducer, e.g. a loudspeaker, arranged close to or in the ear canal, as a unit entirely or partly arranged in the pinna and/or in the ear canal, as a unit, e.g. a vibrator, attached to a fixture implanted into the skull bone, etc. The hearing aid may comprise a single unit or several units communicating (e.g. acoustically, electrically or optically) with each other. The loudspeaker may be arranged in a housing together with other components of the hearing aid, or may be an external unit in itself (possibly in combination with a flexible guiding element, e.g. a dome-like element).

A hearing aid may be adapted to a particular user's needs, e.g. a hearing impairment. A configurable signal processing circuit of the hearing aid may be adapted to apply a frequency and level dependent compressive amplification of an input signal. A customized frequency and level dependent gain (amplification or compression) may be determined in a fitting process by a fitting system based on a user's hearing data, e.g. an audiogram, using a fitting rationale (e.g. adapted to speech). The frequency and level dependent gain may e.g. be embodied in processing parameters, e.g. uploaded to the hearing aid via an interface to a programming device (fitting system), and used by a processing algorithm executed by the configurable signal processing circuit of the hearing aid.

A 'hearing system' refers to a system comprising one or two hearing aids, and a 'binaural hearing system' refers to a system comprising two hearing aids and being adapted to cooperatively provide audible signals to both of the user's ears. Hearing systems or binaural hearing systems may further comprise one or more 'auxiliary devices', which communicate with the hearing aid(s) and affect and/or benefit from the function of the hearing aid(s). Such auxiliary devices may include at least one of a remote control, a remote microphone, an audio gateway device, an entertainment device, e.g. a music player, a wireless communication device, e.g. a mobile phone (such as a smartphone) or a tablet or another device, e.g. comprising a graphical interface. Hearing aids, hearing systems or binaural hearing systems may e.g. be used for compensating for a hearing-impaired person's loss of hearing capability, augmenting or protecting a normal-hearing person's hearing capability and/or conveying electronic audio signals to a person. Hearing aids or hearing systems may e.g. form part of or interact with public-address systems, active ear protection systems, handsfree telephone systems, car audio systems, entertainment (e.g. TV, music playing or karaoke) systems, teleconferencing systems, classroom amplification systems, etc.

The invention is set out in the appended set of claims.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 schematically shows an embodiment of an ear lock for a hearing aid according to the present disclosure,
FIG. 2 schematically shows an embodiment of an ear lock for a hearing aid according to the present disclosure,
FIG. 3 schematically shows an embodiment of an ear lock for a hearing aid according to the present disclosure,
FIG. 4 schematically shows an embodiment of an ear lock for a hearing aid according to the present disclosure,
FIGS. 5A-5D schematically shows an embodiment of an ear lock with a hearing aid according to the present disclosure, and
FIG. 6 schematically shows an embodiment of an ear lock with a hearing aid located in an ear canal according to the present disclosure.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The present application relates to the field of ear locks for hearing aids.

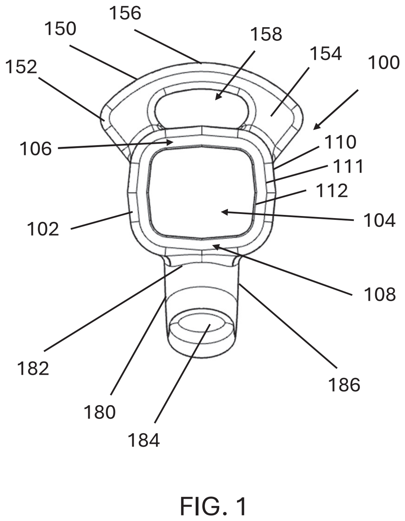

FIG. 1 schematically shows an embodiment of an ear lock for a hearing aid according to the present disclosure. As shown, the ear lock 100 includes a body 102, a first extension 150 and a second extension 180.

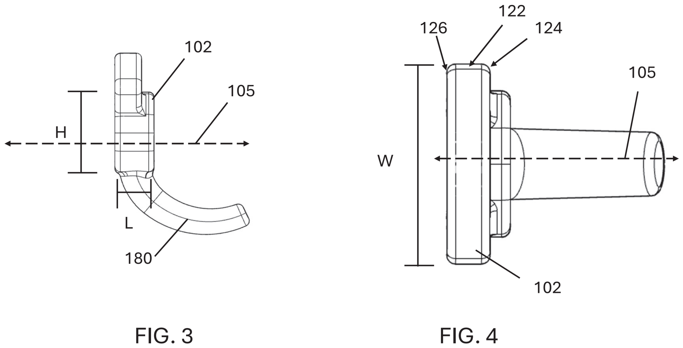

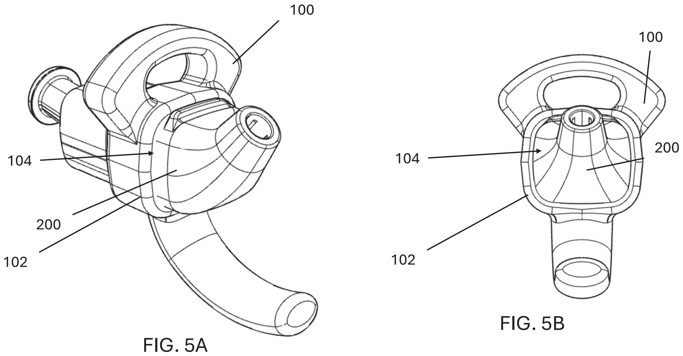

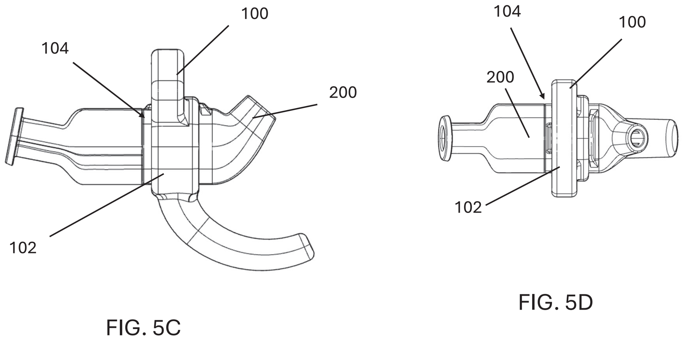

The body 102 includes an aperture 104 extending along a longitudinal axis 105 (shown in FIG. 3) of the body 102. The body is configured to receive an output transducer 200 of a hearing aid within the aperture 104 (shown in FIGS. 5A-5D). The body 102 is formed from an elastic material. This allows the body 102 to stretch to accommodate the output transducer 200 within the aperture 104.

As shown, the body 102 and/or the ear lock 100 includes a first longitudinal end 101 and a second longitudinal 103. These ends 101/103 can define the outer longitudinal boundaries of one or more of the body 102, the ear lock 100, and the aperture 104.

The body 102 can be defined by an inner surface 112 and an outer surface 110. The inner surface 112 and the outer surface 110 can be connected via a connecting surface 111 or via a pair of connecting surfaces 111. The connecting surfaces 111 can define the first longitudinal end 101 and the second longitudinal end 103 of the body 102. The inner surface 112 can define the radial boundaries of the aperture 104.

The first extension 150 is attached to the body 102 on a first side 106 of the body 102 and extending away from the body 102. As shown, the first side 106 can be defined as a first half of the body 102. The first extension 150 is configured to abut a first surface of an ear canal 600 of a user. The first extension 150 can be attached to the body 102 on a first side 106 or a first surface 114A. The first extension 150 extends orthogonal to the longitudinal axis 105.

As shown, the first extension 150 includes a first arm 152 and a second arm 154. The first extension 140 further includes a bridge arm 156. The bridge arm 156 can connect distal ends of the first arm 152 and the second arm 154, thereby forming (e.g., defining) an extension aperture 158. The extension aperture 158 can extend in the same direction (or generally in the same direction) as the aperture 104. The extension aperture 158 can facilitate bending of the first extension 150.

The second extension 180 is attached to the body 102 on a second side 108 of the body 102. The second extension 180 extends away from the body 102. As shown, the second side 108 can be defined as a second half of the body 102. The second extension 180 is configured to abut a second surface of the ear canal 600. The second extension 180 can be attached to the body 102 on a second side 108 or a second surface 114B.

The second extension 180 can be attached to the body 102 at a second proximal end 182 of the second extension 180. The second extension 180 can have a second distal end 184. The second distal end can be spaced radially and longitudinally away from the body 102. The second distal end 184 can be a free end. A second extension body 186 can connect the second proximal end 182 and the second distal end. 184.

The first extension 150 and the second extension 180 can be compressible (e.g., elastic, formed from a compressible material, formed from an elastic material).

The first side 106 is generally opposite on the body 102 from the second side 108.

The body 102 can be formed from a first material and the first extension 150 and the second extension 180 are formed from a second material. For example, the first material can be a polyamide and the second material can be silicon. The first material can be an ester-based thermoplastic polyurethane.

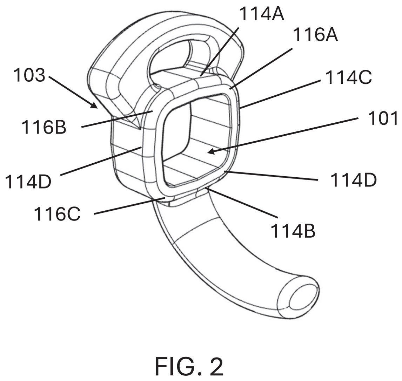

FIG. 2 schematically shows an embodiment of an ear lock for a hearing aid according to the present disclosure. The ear lock 100 of FIG. 2 can include any and/or all of the features discussed with respect to FIG. 1.

As shown in FIG. 2, the outer surface 110 can include first, second, third, and fourth surfaces 114A-114D. The surfaces 114A-114D can be connected via first, second, third, and fourth corners 116A-116D.

FIG. 3 schematically shows an embodiment of an ear lock for a hearing aid according to the present disclosure. The ear lock 100 of FIG. 3 can include any and/or all of the features discussed with respect to FIGS. 1-2.

As shown in FIG. 3, the second extension 180 is curved with respect to the longitudinal axis 105. The second extension 180 extends at least partially away from the longitudinal axis 105 and at least partially in the direction of the longitudinal 105. As shown, the second extension 180 has a concave curve with respect to the longitudinal axis 105.

FIG. 3 further illustrates a length (L) and height (H) of the body 102.

FIG. 4 schematically shows an embodiment of an ear lock for a hearing aid according to the present disclosure. The ear lock 100 of FIG. 4 can include any and/or all of the features discussed with respect to FIGS. 1-3.

FIG. 4 in particular illustrates that the body 102 can include a first section 122 where an outer surface 110 of the body 102 is aligned along the longitudinal axis 105 and a second section 124 where the outer surface 110 curves with respect to the longitudinal axis 105. In other words, the first section 122 can be parallel to the longitudinal axis 105. The body 102 can further include a third section 126. The third section 126 can be a mirror of second section 124. The third section 126 can have a different curve than the second section 124.

FIG. 4 further illustrates a width (W) of the body.

FIGS. 5A-5D schematically shows an embodiment of an ear lock with a hearing aid according to the present disclosure. As shown, the body 102 (e.g., the ear lock 100) is configured to stretch to receive the output transducer 200 within the aperture 104. The output transducer can be a BTE hearing aid output transducer. The output transducer can be a RITE hearing aid output transducer.

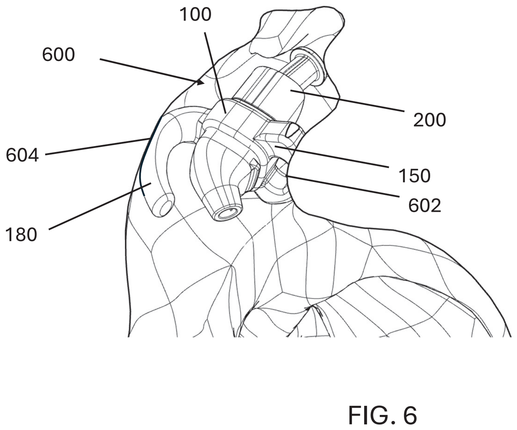

FIG. 6 schematically shows an embodiment of an ear lock 100 with a hearing aid located in an ear canal 600 according to the present disclosure. In particular, the output transducer 200 is within the aperture 104 of the body 102 of the ear lock 100.

As shown, the first extension 150 abuts a first surface 602 of the ear canal 600. The first extension 150 can compress, such as by having the bridge arm 156 compress inwards towards the body 102. The second extension 180 abuts a second surface 604 of the ear canal. The second extension 180 can compress inwards. In certain examples, the second extension 180 is configured to apply a spring effect force on the second surface 504 and is configured to press the first extension 150 into the first surface 602. This can allow the ear lock 100 to main position in the ear canal of the user 100, such as during activity. Further, the second extension 180 is configured to apply an inward force into the ear canal 600, thus preventing outward movement of the ear lock 100, which can in turn prevent the ear lock (and the output transducer) from falling out of the ear canal 600. Moreover, the first extension 150 and the second extension 180 are configured to prevent rotation of the ear lock 100 in the ear canal 600. For example, as the ear lock 100 is compressed into the ear canal 600, the ear lock 100 will fit tightly and therefore will not be rotated.

In certain examples, the ear lock 100 is configured to be centered in the ear canal 600 by a three-point suspension with respect to the first extension 150 and the second extension 180. For example, the first extension 150 can have a first arm 152 and a second arm 154 and the second extension 180 is an arm, thereby providing the three-point suspension.

As shown, the ear lock 100 does not extend out of the ear canal 600 during use. This makes the ear lock 100, and thus the hearing aid as a whole, more discrete and less noticeable.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. An ear lock for maintaining an output transducer of a hearing aid in a user's ear canal, the ear lock comprising:
a body comprising an aperture extending along a longitudinal axis of the body, the body configured to receive an output transducer of a hearing aid within the aperture, wherein the body is formed from an elastic material;
a first extension attached to the body on a first side of the body and extending away from the body, wherein the first extension is configured to abut a first surface of the ear canal; and
a second extension attached to the body on a second side of the body and extending away from the body, wherein the second extension is configured to abut a second surface of the ear canal;
where the first extension comprises a first arm, a second arm, and a bridge arm connecting the first arm and the second arm; and
wherein the second extension is curved with respect to the longitudinal axis, wherein the second extension extends at least partially away from the longitudinal axis and at least partially in the direction of the longitudinal axis.

2. The ear lock of claim 1, wherein the first side is generally opposite on the body from the second side.

3. The ear lock of any one of the preceding claims, wherein the first extension extends orthogonal to the longitudinal axis.

4. The ear lock of any one of the previous claims, wherein the body is configured to stretch to receive the output transducer within the aperture.

5. The ear lock of any one of the previous claims, wherein the body is formed from a first material and the first extension arm and the second extension arm are formed from a second material.

6. The ear lock of claim 5, wherein the first material comprises a polyamide and the second material comprises silicon.

7. The ear lock of claim 5, wherein the first material comprises an ester-based thermoplastic polyurethane and the second material comprises silicon.

8. The ear lock of any one of the previous claims, wherein the body includes a first section where an outer surface of the body is aligned along the longitudinal axis and a second section where the outer surface curves with respect to the longitudinal axis.

9. The ear lock of any one of the previous claims, wherein the ear lock does not extend out of the ear canal during use.

10. The ear lock of any one of the previous claims, wherein the second extension is configured to apply a spring effect force on the second surface and is configured to press the first extension into the first surface.

11. The ear lock of any one of the previous claims, wherein the second extension is configured to apply an inward force into the ear canal.

12. The ear lock of any one of the previous claims, wherein the ear lock is configured to be centered in the ear canal by a three-point suspension with respect to the first extension and the second extension.

13. The ear lock of any one of the previous claims, wherein the first extension and the second extension are configured to prevent rotation of the ear lock in the ear canal.
